# EUROPEAN PATENT APPLICATION

(11) **EP 1 832 250 A1**
(43) Date of publication of application: **12.09.2007**
(21) Application number: 06005010.1
(22) Date of filing: 11.03.2006
(51) Int. Cl.: A61F 2/82

(54) **Stent extractor**

(71) Applicant: Dr. Karel Volenec - ELLA - CS, 500 06 Hradec Kralove (CZ)
(72) Inventor: Petr, Kubena, 500 00 Hradec Kralove (CZ)

(57) **Abstract**

A stent extractor exclusively serves for removing a stent from the esophagus.
It consists of an arresting device serving for grasping implanted stent, and compressing device serving for compressing of the grasped stent inside a lumen thereof. The arresting device is introduced into the esophagus by using an endoscope; the compressing device is put onto the arresting device and introduced under fluoroscopic guidance.

## Description

This invention relates generally to medical devices. More particularly, the field of art into which this invention falls is devices/tools used for removing of implanted stents, more specifically those implanted in gastrointestinal tract.

Stents, i.e. medical devices securing patency of tubular organs, are commonly used in medical practice. They can be divided into two basic groups - a) stents degrading in a patient's body, which pass out naturally and b) stents resistant to degradation, which are either permanent (i.e. remain in situ until a patient's death) or are considered to be removed after their functioning is finished or the reason for being implanted is over.
Some currently available stent delivery systems are documented in patent documents herein:
A removable stent system submitted in Patent Application Publication # US2005/0080480 A1 is based on collapsing one stent end to a smaller diameter. In addition, the invention is intended to remove other implantable devices as well.
Another invention in the field of devices intended for removing of stents is the U.S. patent # 5,474,563 which discloses a stent incl. an apparatus for the insertion/retrieval thereof. A type of the concerned stent is a cardiovascular one; it can be balloon expandable, self-expanding, thermally expandable or expandable by other means. The state of the art also includes e.g. the U.S. patent # 5,749,921 dealing with endoluminal prostheses, particularly stents and stent grafts.

In comparison with the submitted invention, the aforementioned patent documents deal with either a different technical solution of a device designed for removing stents or even describe a device which may even serve for removing different types of stents or possibly other foreign bodies.

A stent extractor of the present invention exclusively serves for removing a stent from the esophagus, or stomach if having been migrated, provided that at least one stent end is equipped with a drawstring.

The stent extractor according to the present invention consists of two substantial parts - an actual arresting device ended with a hook wire which is designed in a question-mark configuration to grasp the drawstring of the stent, and a compressing device serving for compression of the grasped stent inside a lumen thereof so as not to injure surrounding tissue during withdrawing. The stent extractor is specific in the aspect that both aforementioned substantial parts are introduced to the desired place in a patient's body under different guidance and at different times.

The arresting device is introduced to the implanted stent in the esophagus, possibly into the stomach if the stent has migrated, through a working channel of an endoscope; it comprises an elongated tubular member of such a diameter to be accommodated by the working channel of the endoscope and of such a length to reach the stent in a patient's body with its distal end whereas its proximal end sufficiently projects from the endoscope. The elongated tubular member accommodates a core wire leading therethrough which is formed into an arresting hook at the distal end by means of which the stent drawstring is grasped; the core wire is movable in an axial direction being controlled by means of its proximal portion such that if pulled proximally to the maximum, the arresting hook is drawn into the distal portion of the elongated tubular member, which prevents the grasped stent drawstring from slipping therefrom. The core wire is in its proximal portion equipped with an axially movable tightening member, which is also of such a diameter to be accommodated by the working channel of the endoscope, working in the manner of a wedge - if pushed distally, tightly to the proximal end of the elongated tubular member, it wedges the core wire thereinside to prevent it from moving. All these actions are performed under endoscopic guidance.

The endoscope is removed whereas the compressing device is put onto, but not limited to, an auxiliary guide wire, which is fixed to the proximal end of the core wire, and slid distally up to the very distal end of the elongated tubular member inside which the arresting hook holds the stent drawstring. The compressing device comprises a sheath of such a diameter to accommodate the arresting device and of such a length to still sufficiently project from the patient's mouth with its proximal portion even when its distal portion is introduced in the patient's stomach. If the compressing device is pushed further distally, it overlaps the stent drawstring and subsequently leans against the proximal stent end; further distal pushing causes that the stent drawstring purses the stent end to the minimum diameter which allows the sheath to gradually overlap the whole stent lengthwise inside its lumen so as not to injure the tissue during withdrawal.
The whole stent extractor with the stent is then safely removed from a patient's body.
Fig. 1 is a perspective view of the preferred embodiment of the stent extractor according to the present invention.
Fig. 2 is a perspective view of an arresting device of the preferred embodiment of the stent extractor of Fig. 1.
Fig. 3 is a perspective view of a compressing device of the preferred---embodiment of the stent extractor of Fig. 1.
Fig. 4 is a detailed side view of the arresting device of the preferred embodiment of the stent extractor of Fig. 1.
Fig. 5 is a plan as well as side view of a detail of an arresting hook of the preferred embodiment of the stent extractor of Fig. 1.
Fig. 6 is a side view of a detail of the fixation of a core wire and wire members of the preferred embodiment of the stent extractor of Fig. 1.
Fig. 7 is a side as well as front view of a detail of a safety wedge with a pin of the preferred embodiment of the stent extractor of Fig. 1.
Fig. 8 is a detailed schematic side view of the arresting device of the preferred embodiment of the stent extractor of Fig. 1 when the arresting hook is secured against moving by using a safety wedge
Fig. 9 is a partly cross-sectional side view of the course of overlapping a stent by pushing an outer sheath of the compressing device.

A preferred embodiment of the stent extractor of the present invention exclusively serves for removing a stent from the esophagus, or possibly from stomach if having been migrated, provided that at least one stent end is equipped with a drawstring.

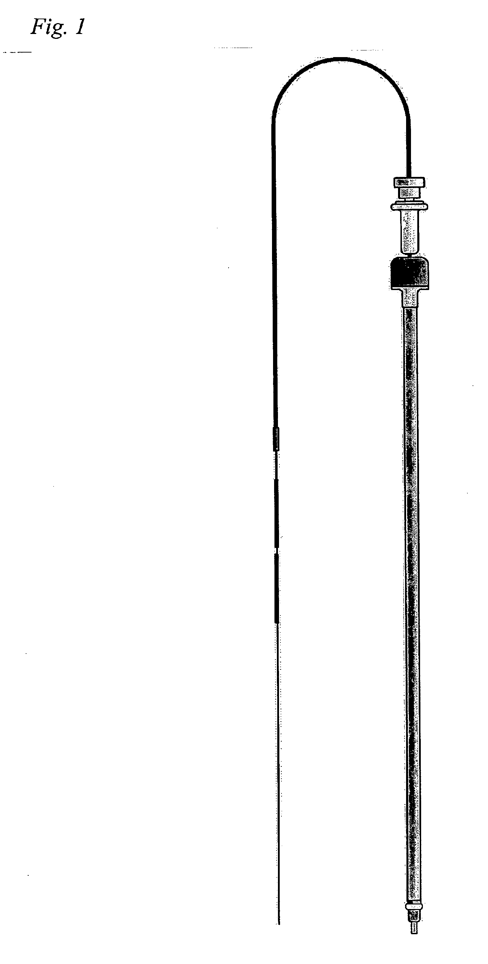

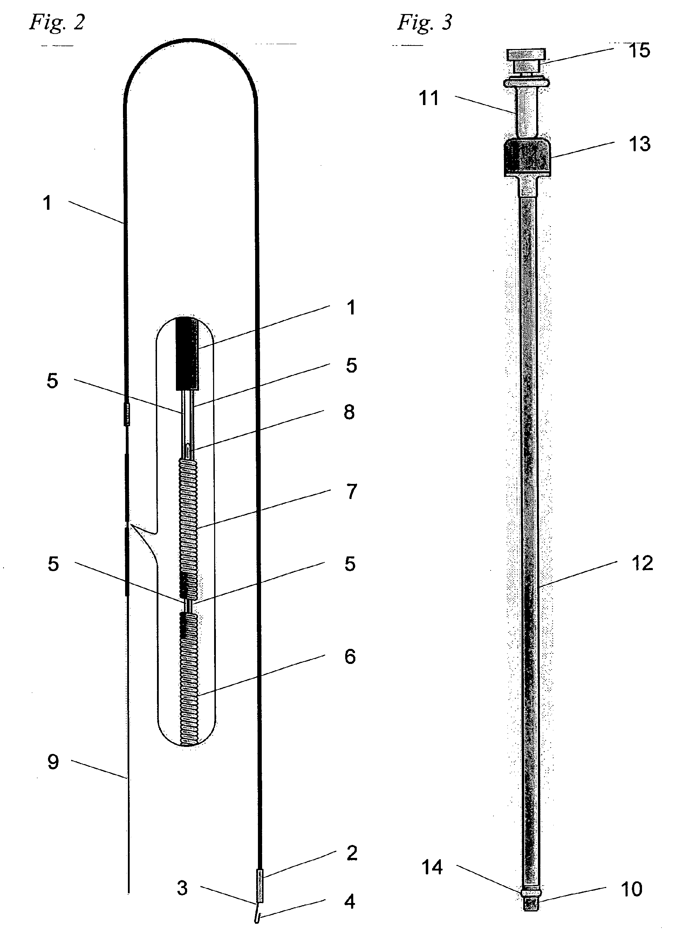

Figures 1 - 9 present a preferred embodiment of the stent extractor; the stent extractor according to the present invention (see Fig. 1) consists of two main parts - an arresting device (shown in Fig. 2) which serves for actual grasping and holding of an implanted stent, and a compressing device (shown in Fig. 3) which serves for compressing of the grasped stent inside a lumen thereof so as not to injure surrounding tissue during withdrawal.

A specificity of the present invention consists in the fact that the arresting device is introduced first under endoscopic guidance (by means of an endoscope) whereas the compressing device is introduced afterwards under fluoroscopic (x-ray) guidance.

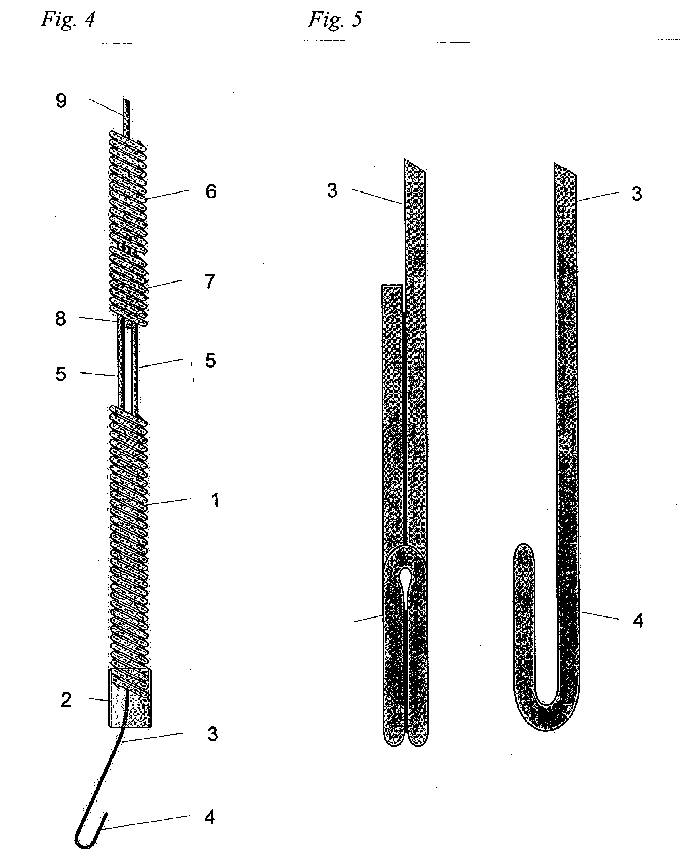

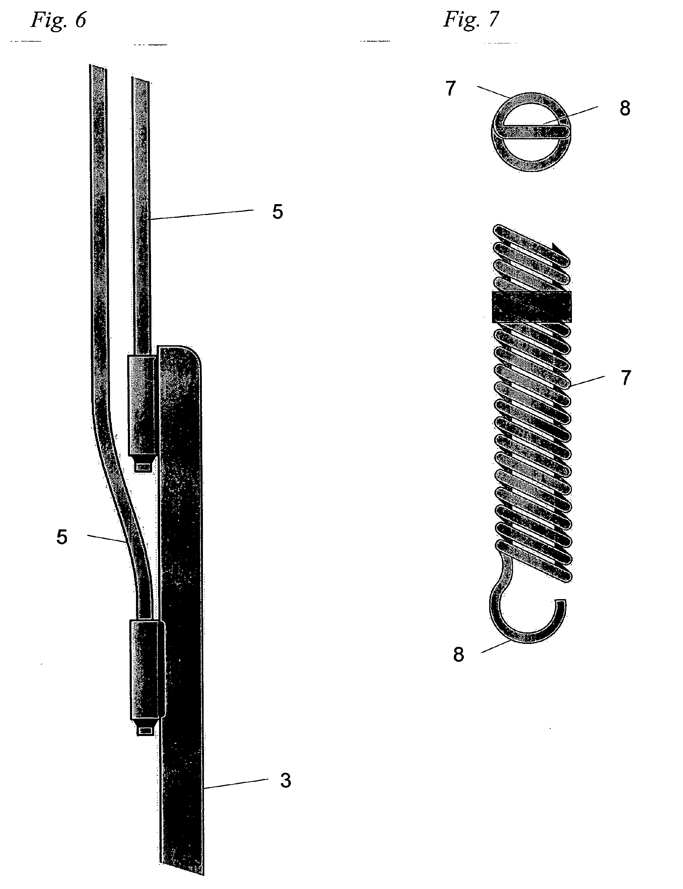

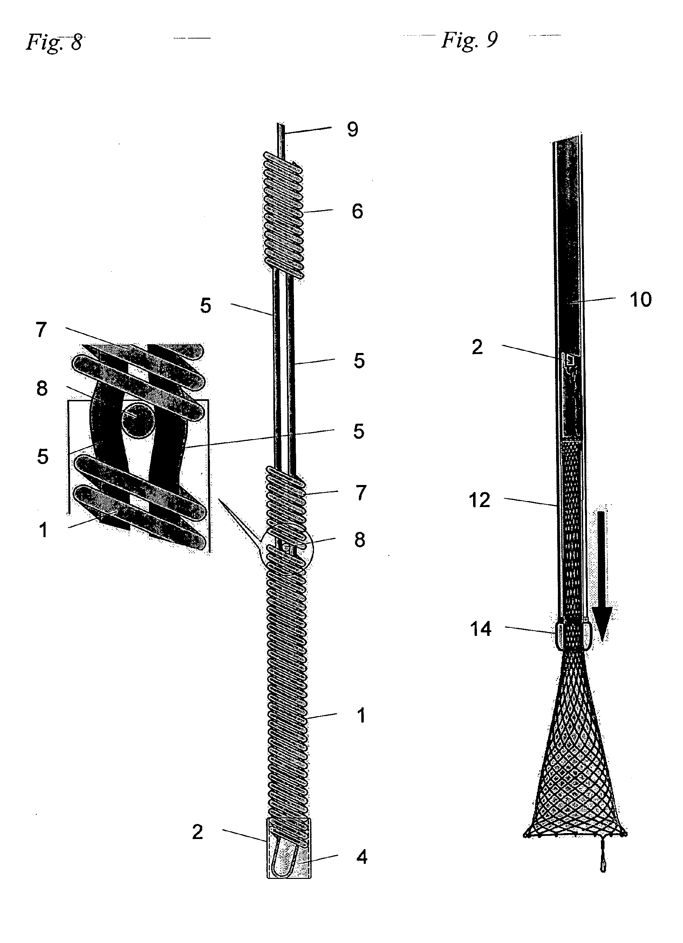

The arresting device, schematically pictured in Fig. 4, is introduced into the esophagus, possibly into the stomach if the stent has migrated, through a working channel of the endoscope; it comprises, but not limited to, a coil spring 1 of such an outer diameter to be accommodated by the working channel of the endoscope and of such a length to reach the stent in the patient's body with its distal portion whereas its proximal portion sufficiently projects from the endoscope. A distal end of the coil spring 1 is equipped with, but not limited to, a tube serving as a lock 2 wherein the stent drawstring is to be secured. The coil spring 1 accommodates a core wire 3 leading therethrough such that the core wire 3 distally reaches the lock 2 and proximally reaches the proximal portion of the coil spring 1 such not to stick out. The distal portion of the core wire 3 is formed into an arresting hook 4 (see the detail in Fig. 5) by means of which the stent drawstring is to be grasped. To the proximal end of the core wire 3 two wire members 5 are firmly fixed (see the detail in Fig. 6), sticking proximally out of the coil spring 1; they are axially directed, arranged to serve as rails. To the proximal end of the wire members 5 a hook control 6 is firmly attached; it is of such a diameter to be accommodated by the working channel of the endoscope as well. The core wire 3 ended with the arresting hook 4, wire members 5, and hook control 6 are movable in axial direction such that if pushed distally to the maximum, the arresting hook 4 is fully drawn out of the lock 2 to grasp the stent drawstring; if pulled proximally to the maximum, the arresting hook 4 is drawn into the lock 2 to secure the grasped stent drawstring from slipping therefrom. A safety wedge 7 is circumferentially disposed on the wire members 5 such that its pin 8 is put therebetween, oriented distally. The safety wedge 7 (see the detail in Fig. 7) is axially movable between the proximal end of the coil spring 1 and distal end of the hook control 6 such that if the safety wedge 7 is pushed distally, tightly to the proximal end of the coil spring 1, its pin 8 wedges the wire members 5 (as shown in Fig. 8) which prevents the arresting hook 4 with grasped stent drawstring from moving.

In addition, the arresting device is equipped with, but not limited to, an auxiliary guide wire 9 which is fastened to the proximal portion of the hook control 6; it serves for introduction of the compressing device.

All the aforementioned actions are to be performed under endoscopic guidance. When the stent drawstring is held inside the lock 2 and prevented from movement, the endoscope can be removed from the patient's body.

The compressing device is put onto the auxiliary guide wire 9 and slid distally up to the lock 2 inside which the arresting hook 4 holds the stent drawstring.

The compressing device comprises an inner sheath 10 with a handle 11 and outer sheath 12 with a handle 13 which are coaxially connected; both sheaths carry a radiopaque ring at the distal end for the reason of fluoroscopic guidance. The inner sheath 10 is of such an inner diameter to accommodate the arresting device and of such a length to still sufficiently project from the patient's mouth with its proximal portion even when its distal portion is introduced in the patient's stomach. The outer sheath 12 is of such an inner diameter to accommodate the inner sheath 10 and of such a length to reach the distal end of the inner sheath 10 distally and the inner sheath handle 11 proximally (as shown in Fig. 3); the outer sheath 12 is distally ended with an atraumatic tip 14 which helps unstick the wall of the implanted stent from that of the esophagus. In addition, the compressing device is equipped with a screw brake 15 which is screwed on the proximal end of the inner sheath 10; if being screwed clockwise, it tightens the proximal end of the inner sheath 10 which prevents the arresting device leading therethrough from moving.

If the compressing device is pushed further distally, its inner sheath 10 overlaps the grasped stent drawstring and subsequently leans against the proximal stent end; further distal pushing against the proximal stent end causes the stent drawstring to elongate and purse the proximal stent end to the minimum diameter simultaneously. The screw brake 15 is then tightened to fix the mutual position of the inner sheath 10 and arresting device leading therethrough. The outer sheath 12 of the compressing device is subsequently pushed further distally, which overlaps first the pursed proximal stent end and then simultaneously overlaps and compresses the whole remaining stent length inside its lumen (see Fig. 9).

The complete stent extractor with the compressed stent inside is then safely removed from the patient's body.

## Claims

1. A stent extractor of the present invention exclusively serving for removing a stent, to which it is introduced through a working channel of an endoscope, from the esophagus, or stomach if having been migrated, provided that at least one stent end is equipped with a drawstring, comprises, but not limited to, a hook wire which is designed in a question-mark configuration to grasp the drawstring of the stent, and, but not limited to, a sheath serving for compression of the grasped stent inside a lumen thereof is **characterized by**:
• an arresting device comprising an elongated tubular member of such a length to reach the stent even in a patient's stomach with its distal end whereas its proximal end sufficiently projects from the endoscope; the elongated tubular member accommodates a core wire (3) leading therethrough which is formed into an arresting hook (4) at the distal end; the core wire (3) is movable in an axial direction being controlled in its proximal portion such that if pulled proximally to the maximum, the arresting hook (4) is drawn into the distal portion of the elongated tubular member; the core wire (3) is in its proximal portion equipped with an axially movable tightening member, which, if pushed distally, tightly to the proximal end of the elongated tubular member, prevents the core wire (3) together with arresting hook (4) from moving; and by
• a compressing device comprising, but not limited to, a sheath of such an inner diameter to accommodate the arresting device together with grasped stent inside a lumen thereof.

2. The stent extractor according to claim 1 **characterized in that** the elongated tubular member comprises a coil spring (1) of such an outer diameter to be accommodated by the working channel of the endoscope and of such a length to reach the stent in the patient's body with its distal portion whereas its proximal portion sufficiently projects from the endoscope; its distal end is equipped with a tube serving as a lock (2) of the arresting hook (4).

3. The stent extractor according to claims 1 and 2 **characterized in that** the coil spring (1) accommodates the core wire (3) leading therethrough such that the core wire (3) ended with the arresting hook (4) distally reaches the lock (2) and proximally reaches the proximal portion of the coil spring (1) such not to stick out; to the proximal end of the core wire (3) two wire members (5) are firmly fixed such to stick proximally out of the coil spring (1), being axially directed, rails-arranged; to the proximal end of the wire members (5) a hook control (6) is firmly fixed, being of such a diameter to be accommodated by the working channel of the endoscope.

4. The stent extractor according to claim 3 **characterized in that** the core wire (3) ended with the arresting hook (4), wire members (5), and hook control (6) are movable in axial direction such that if pushed distally to the maximum, the arresting hook (4) is drawn out of the lock (2), if pulled proximally to the maximum, the arresting hook (4) is drawn into the lock (2).

5. The stent extractor according to claims 3 and 4 **characterized in that** a safety wedge (7) with a distally oriented pin (8) is circumferentially disposed on the wire members (5) such that the pin (8) is put therebetween; the safety wedge (7) is axially movable between the proximal end of the coil spring (1) and distal end of the hook control (6) such that if the safety wedge (7) is pushed distally, tightly to the proximal end of the coil spring (1), its pin (8) wedges the wire members (5) inside the coil spring (1) to prevent the arresting hook (4) from moving.

6. The stent extractor according to claim 1 **characterized in that** an auxiliary guide wire (9) is firmly fixed to the proximal portion of the hook control (6) by means of which the compressing device is put onto the arresting device and introduced into a patient's body.

7. The stent extractor according to claim 1 **characterized in that** the arresting device is introduced first under endoscopic guidance into a patient's body whereas the compressing device is introduced afterwards under fluoroscopic guidance.

8. The stent extractor according to claim 1 **characterized in that** the compressing device comprises
• an inner sheath (10) with a handle (11) proximally and a radiopaque ring distally, and
• an outer sheath (12) with a handle (13) proximally, a radiopaque ring and an atraumatic tip (14) distally
which are coaxially connected and coaxially movable such that the inner sheath (10) is of such an inner diameter to accommodate the arresting device and stent drawstring but fails to accommodate the stent end itself, and of such a length to project from the patient's mouth with its proximal portion even when its distal portion is introduced in the patient's stomach; the outer sheath (12) is of such an inner diameter to accommodate the inner sheath (10) and the stent in a compressed condition, and of such a length to reach the distal end of the inner sheath (10) distally and inner sheath handle (11) proximally.

9. The stent extractor according to claim 8 **characterized in that** the inner sheath (10) is in its proximal portion equipped with a screw brake (15); if screwed clockwise, it tightens the proximal end of the inner sheath (10), which prevents the arresting device leading therethrough from moving.
